# EUROPÄISCHE PATENTANMELDUNG

(11) **EP 4 837 560 A1**
(43) Veröffentlichungstag der Anmeldung: **11.11.2026**
(21) Anmeldenummer: 26166168.0
(22) Anmeldetag: 19.03.2026
(51) Int. Cl.: A61K 8/41, A61K 8/49, A61Q 5/10

(54) **MEHRKOMPONENTEN-VERPACKUNGSEINHEIT ZUR FÄRBUNG VON KERATINISCHEN FASERN UMFASSEND OXIDATIONSFARBSTOFFVORPRODUKTE UND ALIPHATISCHE DI- ODER POLYAMINOVERBINDUNGEN IN GETRENNTEN CONTAINERN**

(30) Priorität: 07.05.2025 DE 102025117562
(71) Anmelder: Henkel AG & Co. KGaA, 40589 Düsseldorf (DE)
(72) Erfinder: Nowottny, Marc, 41189 Mönchengladbach (DE); Jaiser, Phillip, 40764 Langenfeld (DE); Mandal, Ranju Prasad, 40591 Düsseldorf (DE); Lechner, Torsten, 40764 Langenfeld (DE); Chardon, Gaelle, 69720 Saint-Bonnet-de-Mure (FR)

(57) **Zusammenfassung**

Gegenstand der vorliegenden Erfindung ist eine Mehrkomponenten-Verpackungseinheit (Kit-ofparts) zum Färben von keratinischen Fasern, insbesondere menschlichen Haaren, umfassend getrennt voneinander konfektioniert
- einen ersten Container mit einer ersten Zusammensetzung (A), und
- einen zweiten Container mit einer zweiten Zusammensetzung (B), wobei
- die Zusammensetzung (A) in einem wasserhaltigen kosmetischen Träger mindestens ein Oxidationsfarbstoffvorprodukt enthält und einen pH-Wert von 4,0 bis 8,0 besitzt, und
- die Zusammensetzung (B) mindestens eine aliphatische Di- oder Polyaminoverbindung (DA) der Formel (I) und/oder deren Salz enthält, 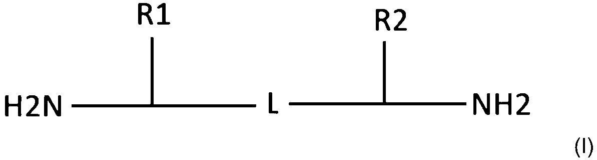 wobei
R1 und R2
unabhängig voneinander für ein Wasserstoffatom, eine C₁-C₆-Alkylgruppe oder eine Hydroxy-C₁-C₆-alkylgruppe stehen, oder aber R1 und R2 bilden zusammen mit den beiden Kohlenstoffatomen, an die sie gebunden sind, und dem Linker L einen 5-, 6- oder 7-gliedrigen, gesättigten, carbozyklischen oder heterozyklischen Ring,
L
für eine direkte Bindung oder für eine gesättigte oder ungesättigte, lineare oder verzweigte C₁-C₁₂-Alkylengruppe steht, die gegebenenfalls auch mit einer oder mehreren Hydroxygruppen substituiert und/oder durch eine oder mehrere Sauerstoffatome und/oder durch eine oder mehrere Gruppierungen -NR3- unterbrochen sein kann, und
R3
für ein Wasserstoffatom, eine C₁-C₆-Alkylgruppe, eine Hydroxy-C₁-C₆-alkylgruppe oder eine Amino-C₁-C₆-Alkylgruppe steht.

## Beschreibung

Die vorliegende Anmeldung liegt auf dem Gebiet der Kosmetik und betrifft eine Mehrkomponenten-Verpackungseinheit (Kit-of-parts) zum Färben von keratinischen Fasern, insbesondere menschlichen Haaren, welche getrennt voneinander konfektioniert in zwei verschiedenen Containern die Zusammensetzungen (A) und (B) umfasst. Die Zusammensetzung (A) enthält in einem wasserhaltigen kosmetischen Träger mindestens ein Oxidationsfarbstoffvorprodukt und besitzt pH-Wert von 4,0 bis 8,0. Die Zusammensetzung (B) ist gekennzeichnet durch ihren Gehalt an mindestens einer aliphatische Di- oder Polyaminoverbindung der Formel (I).

Ein zweiter Gegenstand der vorliegenden Anmeldung ist ein anwendungsbereites Färbemittel, das durch Vermischen der beiden Zusammensetzungen (A) und (B) erhalten wird.

Ein dritter Gegenstand der vorliegenden Anmeldung betrifft ein Verfahren zur durch Luftsauerstoff induzierten Färbung keratinhaltiger Fasern, insbesondere menschlicher Haare, welches das Bereitstellen der beiden Zusammensetzungen (A) und (B), ihr Vermischen und danach das Auftragen und Einwirken der Mischung aus (A) plus (B) auf die keratinischen Fasern umfasst.

Die Veränderung von Form und Farbe von keratinischen Fasern, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Ein weiteres oxidatives Färbeverfahren kann mit Luftsauerstoff als einzigem Oxidationsmittel durchgeführt werden. Im Rahmen dieses Verfahrens werden Oxidationsfarbstoffvorprodukte, die ein geringeres Redoxpotential als die üblichen Kuppler und Entwickler besitzen, auf die Haare aufgetragen. Da die Farbstoffvorstufen dieser Klasse extrem oxidationsempfindlich sind und bereits an der Luft oxidiert werden können, werden Farbstoffvorstufen dieses Typs auch als selbstoxidierende Farbstoffe (SOF) bezeichnet. Nach dem Auftragen auf die keratinischen Fasern werden die Farbstoffe üblicherweise in alkalischem Milieu durch Luftsauerstoff oxidiert und bilden in der Folge tieffarbige Kondensations- bzw. Kupplungsprodukte auf dem Haar aus. Besonders bekannte Vertreter dieser Farbstoffklasse sind zum Beispiel 5,6-Dihydroxyindolin oder 5,6-Dihydroxyindol. Da Farbstoffe auf Indol- bzw. Indolin-Basis auch die Vorstufen des natürlichen Haarfarbstoffes Melanin darstellen, können sie Anwendern mit ergrauten Haaren ihre natürliche Haarfarbe wieder zurückgeben.

Die oxidative Kupplungsreaktion findet üblicherweise bei alkalischen pH-Werten statt, so dass die anwendungsbereiten Färbemittel meist auf einen pH-Wert zwischen 9,5 und 10,5 eingestellt werden. In der Druckschrift WO 93/09759 A1 wird zur Einstellung eines alkalischen pH-Wertes von 9,5 beispielsweise Ammoniak eingesetzt.

Aus der Druckschrift EP 1254650 B1 ist die Verwendung einer Kombination von 5,6-Dihydroxyindolin mit aliphatischen Monoaminoverbindungen wie Monoethanolamin, Isopropanolamin, Butylamin der Benzylamin bekannt.

In der Druckschrift WO 99/66890 A1 werden Haarfärbemittel enthaltend 5,6-Dihydroxyindolin und Aminosäuren oder Oligopeptide beschrieben.

In Reihenversuchen mit verschiedenen pH-Werten hat sich gezeigt, dass die selbstoxidierenden Farbstoffe unter Einwirkung des Luftsauerstoffs ein umso intensiveres Farbergebnis liefern, je alkalischer das auf die Haare applizierte Färbemittel ist. Alkalisierungsmittel lassen das Haar aufquellen und lockern die Wasserstoffbrücken im Haarkeratin, wodurch das Haar auch aufnahmefähiger gemacht wird. Gleichzeitig ist mit dem Aufquellen des Haares jedoch auch eine Schädigung verbunden, wobei das Ausmaß der Schädigung mit steigendem pH-Wert weiter zunimmt. Eine Färbung mit selbstoxidierenden Farbstoffen, die sehr intensive Färbungen liefert, hierbei aber die durch hohe pH-Werte hervorgerufene Schädigung vermeidet, war bislang aus dem Stand der Technik nicht bekannt.

Die Aufgabe der vorliegenden Anmeldung bestand darin, einen Kit-of-Parts bzw. ein Verfahren zur Färbung von keratinischen Fasern auf der Grundlage von selbstoxidierenden Farbstoffen zu finden, das mit Luftsauerstoff als einzigem Oxidationsmittel funktioniert und einen schnellen und vollständigen Farbaufbau gewährleistet. Hierbei sollte die gewünschte Farbnuance bereits nach einmaliger Anwendung und möglichst direkt nach Abspülen des Färbemittels sichtbar sein. Die keratinischen Fasern sollten in möglichst dunklen und intensiven Farben gefärbt werden können, ohne dass die Haare während der Anwendung alkalibedingt zu stark aufquellen oder geschädigt werden.

Überraschenderweise hat sich nun gezeigt, dass die durch Luftsauerstoff induzierte Farbstoffbildungsreaktion von Oxidationsfarbstoffvorprodukten, insbesondere von selbstoxidierenden Farbstoffen, in Anwesenheit von bestimmten aliphatischen Di- oder Polyaminoverbindungen der Formel (I) auch bei niedrigeren pH-Werten schnell und vollständig abläuft.

Ein erster Gegenstand der vorliegenden Anmeldung ist daher eine Mehrkomponenten-Verpackungseinheit (Kit-of-parts) zum Färben von keratinischen Fasern, insbesondere menschlichen Haaren, umfassend getrennt voneinander konfektioniert
- einen ersten Container mit einer ersten Zusammensetzung (A), und
- einen zweiten Container mit einer zweiten Zusammensetzung (B), wobei
- die Zusammensetzung (A) in einem wasserhaltigen kosmetischen Träger mindestens ein Oxidationsfarbstoffvorprodukt enthält und einen pH-Wert von 4,0 bis 8,0 besitzt, und
- die Zusammensetzung (B) mindestens eine aliphatische Di- oder Polyaminoverbindung (DA) der Formel (I) und/oder deren Salz enthält, 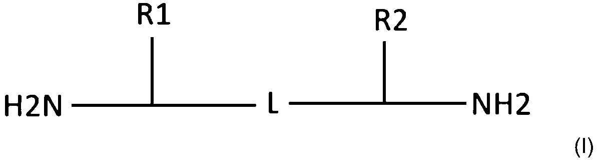
wobei
- R1 und R2: unabhängig voneinander für ein Wasserstoffatom, eine C₁-C₆-Alkylgruppe oder eine Hydroxy-C₁-C₆-alkylgruppe stehen, oder aber R1 und R2 bilden zusammen mit den beiden Kohlenstoffatomen, an die sie gebunden sind, und dem Linker L einen 5-, 6- oder 7-gliedrigen, gesättigten, carbozyklischen oder heterozyklischen Ring,
- L: für eine direkte Bindung oder für eine gesättigte oder ungesättigte, lineare oder verzweigte C₁-C₁₂-Alkylengruppe steht, die gegebenenfalls auch mit einer oder mehreren Hydroxygruppen substituiert und/oder durch eine oder mehrere Sauerstoffatome und/oder durch eine oder mehrere Gruppierungen -NR3- unterbrochen sein kann, und
- R3: für ein Wasserstoffatom, eine C₁-C₆-Alkylgruppe, eine Hydroxy-C₁-C₆-alkylgruppe oder eine Amino-C₁-C₆-Alkylgruppe steht.

Für die Färbung wird durch Vermischen der Zusammensetzungen (A) und (B) das anwendungsbereite Färbemittel hergestellt. Da die Zusammensetzung (A) auf einen für luftoxidative Färbemittel niedrigen pH-Wertebereich von 4,0 bis 8,0 eingestellt ist, hat auch die aus (A) und (B) hergestellte Mischung einen vergleichsweise niedrigen pH-Wert. Überraschenderweise hat sich herausgestellt, dass die mit dem Kit-of-parts realisierbaren Färbungen trotz des niedrigen pH-Wertes besonders dunkel und intensiv sind.

Darüber hinaus hat sich herausgestellt, dass durch die Anwendung der Kombination aus Oxidationsfarbstoffvorprodukt und aliphatischer Di- oder Polyaminoverbindung (DA) der Formel (I) auf Haaren eine stark beschleunigte Farbstoffbildung stattfindet. So konnte eine Nuance, die sich normalerweise ohne Verwendung des Di-/Polyamins (DA) über Tage entwickelt, mit dem Di-bzw. Polyamin (DA) bereits eine Stunde nach der Färbung in ihrer endgültigen Farbtiefe erhalten werden. Aufgrund des bei der Färbung vorliegenden niedrigen pH-Wertes konnten die durch Alkalität hervorgerufenen Haarschädigungen stark reduziert oder sogar ganz vermieden werden.

### Keratinische Fasern

Unter keratinischen Fasern sind Haare, Wolle und Pelze zu verstehen. Ganz besonders bevorzugt wird unter keratinischen Fasern menschliche Haare verstanden.

### Mehrkomponenten-Verpackungseinheit

Die erfindungsgemäße Mehrkomponenten-Verpackungseinheit (Kit-of-parts) umfasst in getrennten Containern die beiden Zusammensetzungen (A) und (B).

Als Container können beispielsweise eine Tube, eine Flasche, eine Dose, ein Becher, ein Sachet oder auch ein anderes Behältnis verwendet werden, das für die Anwendung auch mit einem Applikator versehen werden kann.

Erfindungsgemäß umfasst der Kit-of-Parts mindestens zwei Container. Optional können aber auch noch ein oder mehrere weitere Container umfasst sein. Beispielsweise kann der Kit-of-Parts auch noch mindestens eine weitere getrennt konfektionierte Zusammensetzung (C) in einem dritten Container enthalten, bei der es sich beispielsweise um einen Conditioner ein besonders gut geeignetes Shampoo handeln kann.

### Zusammensetzung (A) im ersten Container

Im ersten Container befindet sich die Zusammensetzung (A), welche ein oder mehrere Oxidationsfarbstoffvorprodukte enthält und einen pH-Wert von 4,0 bis 8,0 besitzt. Damit pH-Wert auf den erfindungsgemäßen Bereich eingestellt werden kann, enthält die Zusammensetzung (A) das oder die Oxidationsfarbstoffvorprodukte in einem kosmetischen Träger, der Wasser umfasst.

Prinzipiell kann die Zusammensetzung (A) ein Oxidationsfarbstoffvorprodukt vom Entwicklertyp und/oder vom Kupplertyp enthalten.

Typische Oxidationsfarbstoffvorprodukte vom Entwicklertyp aus der Gruppe der p-Phenylendiamine sind beispielsweise p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-Methoxymethyl-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Als gut geeignete zweikernige Entwicklerkomponenten können beispielsweise eingesetzt werden: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Als Entwickler aus der Gruppe der p-Aminophenole können beispielsweise genannt werden: p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie deren Salze enthält.

Auch heterozyklische Verbindungen können als Oxidationsfarbstoffe vom Entwicklertyp eingesetzt werden, darunter fallen zum Beispiel 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol sowie deren Salze enthält.

Geeignete Oxidationsfarbstoffen vom Kupplertyp sind beispielsweise 2-Methylresorcin, 4-Chlorresorcin, Resorcin, 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diamino-phenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 1-Amino-3-bis-(2-hydroxyethyl)aminobenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 6-Hydroxybenzomorpholin und deren physiologisch verträglichen Salze.

Der erfindungsgemäße Kit-of-parts eignet sich ganz besonders gut zur luftoxidativen Färbung. Bei einem Verfahren zur durch Luftsauerstoff induzierten Färbung keratinischer Fasern, insbesondere menschlicher Haare, wird mindestens ein selbstoxidierender Farbstoff (SOF) in Kombination mit mindestens einer aliphatischen Di- oder Polyaminoverbindung (DA) der Formel (I) auf den keratinischen Fasern angewendet.

Unter einer Färbung, die durch Luftsauerstoff induziert ist, wird im Sinne der vorliegenden Anmeldung verstanden, dass der in der Luft enthaltene Sauerstoff als einziges Oxidationsmittel zur Anwendung kommt.

Bei den selbstoxidierenden Farbstoffen handelt es sich um aromatische Verbindungen mit mehr als zwei Substituenten am Ring, die ein sehr geringes Redoxpotential besitzen und daher bereits an der Luft oxidiert werden.

Um das Redoxpotential zu erniedrigen, trägt der aromatische Ringe des selbstoxidierenden Farbstoffes (SOF) mehrere Donor-Substituenten wie beispielsweise eine Hydroxygruppe, eine C₁-C₆-Alkoxygruppe, eine Aminogruppe, eine C₁-C₆-Alkylaminogruppe, eine Di-C₁-C₆-Alkylaminogruppe oder auch eine C₁-C₆-Alkylgruppe oder C₁-C₆-Alkylengruppe.

Ein selbstoxidierender Farbstoff (SOF) im Sinne der vorliegenden Anmeldung ist daher ein Oxidationsfarbstoffvorprodukt, das einen aromatischen Ring, insbesondere einen Benzolring, von dem mindestens drei direkte Bindungen zu einem oder mehreren Sauerstoffatomen und/oder Stickstoffatomen ausgehen. Das bzw. die Sauerstoffatome und/oder Stickstoffatome sind Teil eines Substituenten, bei dem es sich um eine Hydroxygruppe, eine C₁-C₆-Alkoxygruppe, eine Aminogruppe, eine C₁-C₆-Alkylaminogruppe oder eine Di C₁-C₆-Alkylaminogruppe handelt. Der aromatische Ring von 5,6-Dihydroxyindolin trägt beispielsweise zwei Hydroxygruppen und besitzt eine weitere direkte Bindung zu einem Stickstoffatom, das an eine C₂-Alkylengruppe gebunden ist, die wiederum mit dem Stickstoffatom und dem aromatischen Ring einen gesättigten 5-Ring ausbildet.

Auch Indolderivate mit einem oder mehreren Hydroxy- und/oder Aminogruppen gehören zu den selbstoxidierenden Farbstoffen (SOF). Da Indol ein besonders elektronenreicher Heteroaromat ist, werden schon die Indolderivate leicht durch Luftsauerstoff oxidiert, die mit mindestens einem starken Donor-Substituenten wie beispielsweise einer Hydroxygruppe und/oder einer Aminogruppe substituiert sind.

Besonders gut geeignete selbstoxidierende Farbstoffe können beispielsweise ausgewählt werden aus der Gruppe, die gebildet wird aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, das 6-Aminoindolin, 4-Aminoindolin, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol, 1,2,4-Trihydroxybenzol, Aminoresorcin, Aminohydrochinon, Diaminophenolen und 3,4,5-Trihydroxybenzoesäure.

In einer besonders bevorzugten Ausführungsform ist eine erfindungsgemäße Mehrkomponenten-Verpackungseinheit deshalb dadurch gekennzeichnet, dass die Zusammensetzung (A) als Oxidationsfarbstoffvorprodukt mindestens einen selbstoxidierenden Farbstoff (SOF) enthält, der ausgewählt ist aus der Gruppe aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin, 4-Aminoindolin, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol, 1,2,4-Trihydroxybenzol, Aminoresorcin, Aminohydrochinon, Diaminophenolen, 3,4,5-Trihydroxybenzoesäure sowie den physiologisch verträglichen Salzen dieser Verbindungen.

In einer besonders bevorzugten Ausführungsform ist eine erfindungsgemäße Mehrkomponenten-Verpackungseinheit auch dadurch gekennzeichnet, dass die Zusammensetzung (A) mindestens einen selbstoxidierenden Farbstoff (SOF) enthält, der ausgewählt ist aus der Gruppe aus 5,6-Dihydroxyindolin, N-Methyl-5,6-dihydroxy-indolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin, 4-Aminoindolin, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol, 1,2,4-Trihydroxybenzol, Aminoresorcin, Aminohydrochinon, Diaminophenolen, 3,4,5-Trihydroxybenzoesäure sowie den physiologisch verträglichen Salzen dieser Verbindungen.

In einer ganz besonders bevorzugten Ausführungsform ist eine erfindungsgemäße Mehrkomponenten-Verpackungseinheit dadurch gekennzeichnet, dass der selbstoxidierende Farbstoff (SOF) ausgewählt ist aus der Gruppe aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 1,2,4-Trihydroxybenzol, Aminoresorcin. Aminohydrochinon, Diaminophenolen sowie den physiologisch verträglichen Salzen dieser Verbindungen.

Die physiologisch verträglichen Salze der selbstoxidierenden Farbstoffe sind Salze, die zum Einsatz im kosmetischen Mittel (d.h. zur Anwendung auf dem menschlichen Haar und der menschlichen Haut) geeignet sind. Besonders gut geeignete physiologisch verträgliche Salze sind zum Beispiel die Hydrobromide, die Hydrochloride, die Hydrogensulfate und die Sulfate der Verbindungen.

Am allermeisten bevorzugt ist 5,6-Dihydroxyindolin, das ganz besonders bevorzugt in Form seines Hydrobromids eingesetzt wird.

Die Zusammensetzung (A) enthält das oder die Oxidationsfarbstoffvorprodukte, insbesondere die selbstoxidierenden Farbstoffe (SOF), bevorzugt in bestimmten Mengenbereichen. Besonders gute Ergebnisse wurden erhalten, wenn die Zusammensetzung (A) - bezogen auf das Gesamtgewicht der Zusammensetzung (A) - einen oder mehrere Oxidationsfarbstoffvorprodukte, besonders bevorzugt einen oder mehrere selbstoxidierende Farbstoffe (SOF), in einer Gesamtmenge von 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% enthielt.

In einer besonders bevorzugten Ausführungsform ist eine erfindungsgemäße Mehrkomponenten-Verpackungseinheit dadurch gekennzeichnet, dass die Zusammensetzung (A) - bezogen auf das Gesamtgewicht der Zusammensetzung (A) - einen oder mehrere Oxidationsfarbstoffvorprodukte, besonders bevorzugt einen oder mehrere selbstoxidierende Farbstoffe (SOF), in einer Gesamtmenge von 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% enthält.

Die Oxidationsfarbstoffvorprodukte bzw. die selbstoxidierenden Farbstoffe (SOF) sind in einem wasserhaltigen kosmetischen Träger in der Zusammensetzung (A) enthalten, bei dem es sich zum Beispiel um einen geeigneten wässrigen oder wässrig-alkoholischen Träger handeln kann. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Der pH-Wert der Zusammensetzung (A) liegt im Bereich von 4,0 bis 8,0, d.h. der pH-Wert ist sauer bis leicht alkalisch. Ein bei der Färbung auftretende Haarschädigung konnte jedoch besonders dann stark reduziert bzw. vollständig vermieden werden, wenn der pH-Wert der Zusammensetzung auf einen Bereich von 4,3 bis 7,5, bevorzugt von 4,6 bis 7,3, weiter bevorzugt von 4,9 bis 7,0 und ganz besonders bevorzugt von 5,1 bis 6,5 eingestellt wurde. Mit Zusammensetzungen (A), die auf einen pH-Wert im Bereich von 5,1 bis 6,5 eingestellt waren, konnten Ausfärbungen mit besonders hoher Farbintensität bei gleichzeitig besonders geringer Haarschädigung erzielt werden.

In einer besonders bevorzugten Ausführungsform ist eine erfindungsgemäße Mehrkomponenten-Verpackungseinheit deshalb dadurch gekennzeichnet, dass die Zusammensetzung (A) einen pH-Wert von 4,3 bis 7,5, bevorzugt von 4,6 bis 7,3, weiter bevorzugt von 4,9 bis 7,0 und ganz besonders bevorzugt von 5,1 bis 6,5 besitzt.

Bei den pH-Werten im Sinne der vorliegenden Erfindung handelt es sich um pH-Werte, die bei einer Temperatur von 22 °C gemessen wurden. Zur Messung des pH-Wertes eignet sich beispielsweise ein Glaselektrode, die in Form einer pH-Einstabmesskette im Handel käuflich erworben werden kann.

Zur Einstellung der erfindungsgemäßen und besonders bevorzugten pH-Wertebereiche können organische und/oder anorganische Säuren verwendet werden.

Hierbei haben sich bestimmte Säuren zur Einstellung des gewünschten pH-Wertes als ganz besonders gut geeignet erwiesen. Diese Säuren können beispielsweise ausgewählt werden aus der Gruppe aus Ascorbinsäure, Zitronensäure, Weinsäure, Äpfelsäure, Milchsäure, Essigsäure, Methansulfonsäure, Benzoesäure, Malonsäure, Oxalsäure, 1-Hydroxyethan-1,1-diphosphonsäure, Schwefelsäure, Salzsäure und Phosphorsäure.

Als ganz besonders gut geeignet hat sich Ascorbinsäure erwiesen, da Ascorbinsäure sowohl den gewünschten pH-Wert einstellt als auch eine antioxidative Wirkung besitzt, so dass durch ihre Anwesenheit ein vorzeitiges Abreagieren des selbstoxidierenden Farbstoffs (SOF) der gelagerten, im noch verschlossenen Container befindlichen Zusammensetzung (A) effektiv vermieden werden kann.

In einer besonders bevorzugten Ausführungsform ist eine erfindungsgemäße Mehrkomponenten-Verpackungseinheit deshalb dadurch gekennzeichnet, dass die Zusammensetzung (A) mindestens eine Säure enthält, die ausgewählt ist aus der Gruppe aus organischen und/oder anorganischen Säuren, besonders bevorzugt aus der Gruppe aus organischen Säuren, ganz besonders bevorzugt Ascorbinsäure.

### Zweiter Container mit der Zusammensetzung (B)

Getrennt konfektioniert in einem zweiten Container umfasst die erfindungsgemäße Mehrkomponenten-Verpackungseinheit die Zusammensetzung (B).

Die Zusammensetzung (B) enthält mindestens eine aliphatische Di- oder Polyaminoverbindung (DA) der Formel (I) und/oder deren Salz, 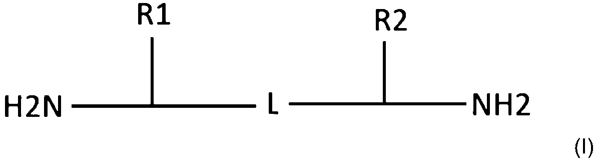 wobei
- R1 und R2: unabhängig voneinander für ein Wasserstoffatom, eine C₁-C₆-Alkylgruppe oder eine Hydroxy-C₁-C₆-alkylgruppe stehen, oder aber R1 und R2 bilden zusammen mit den beiden Kohlenstoffatomen, an die sie gebunden sind, und dem Linker L einen 5-, 6- oder 7-gliedrigen, gesättigten, carbozyklischen oder heterozyklischen Ring,
- L: für eine direkte Bindung oder für eine gesättigte oder ungesättigte, lineare oder verzweigte C₁-C₁₂-Alkylengruppe steht, die gegebenenfalls auch mit einer oder mehreren Hydroxygruppen substituiert und/oder durch eine oder mehrere Sauerstoffatome und/oder durch eine oder mehrere Gruppierungen -NR3- unterbrochen sein kann, und
- R3: für ein Wasserstoffatom, eine C₁-C₆-Alkylgruppe, eine Hydroxy-C₁-C₆-alkylgruppe oder eine Amino-C₁-C₆-Alkylgruppe steht.

Die Di- oder Polyaminoverbindung (DA) der Formel (I) beschleunigen die durch Luftsauerstoff induzierte Oxidation der selbstoxidierenden Farbstoffe (SOF), so dass die Entwicklung der Farbe, die ohne Verwendung der erfindungsgemäßen Di- oder Polyamine verhältnismäßig langsam stattfindet, stark beschleunigt werden kann.

Wie vergleichende Versuche gezeigt haben, wird auch die Kupplungsgeschwindigkeit zum Farbstoff erhöht, so dass die finale Farbentwicklung, auf die der Anwender bei Anwendung eines Produktes ohne Di- bzw. Polyamine üblicherweise mehrere Tage warten muss, soweit beschleunigt werden konnte, dass die Farbentwicklung schon bei Abspülen bzw. spätestens eine Stunde nach Abspülen des Färbemittels vollständig ist.

Die Salze der aliphatische Di- oder Polyaminoverbindung (DA) der Formel (I) sind bevorzugt physiologisch verträglichen Salze, die zum Einsatz im kosmetischen Mittel (d.h. zur Anwendung auf dem menschlichen Haar und der menschlichen Haut) geeignet sind. Besonders gut geeignete physiologisch verträgliche Salze sind zum Beispiel die Hydrochloride, die Hydrobromide, die Hydrogensulfate und die Sulfate der Verbindungen der Formel (I).

Die erfindungsgemäßen Di- oder Polyaminoverbindungen (DA) der Formel (I) sind aliphatisch, was bedeutet, dass ihre Struktur keinen aromatischen Ring umfasst.

Die Di- oder Polyaminoverbindungen (DA) besitzen die Formel H₂N-CHR₁-L-CHR₂-NH₂.

Die zur linken Aminogruppe benachbarte CH-Gruppe trägt den Rest R1. Der Rest R1 kann für ein Wasserstoffatom, eine C₁-C₆-Alkylgruppe oder eine Hydroxy-C₁-C₆-alkylgruppe stehen.

Beispiele für eine C₁-C₆-Alkylgruppe sind die Methylgruppe, die Ethylgruppe, die Propylgruppe, die Butylgruppe, die Pentylgruppe und die Hexylgruppe. Beispielhaft für eine gut geeignete Hydroxy-C1-C6-alkylgruppe können die 2-Hydroxyethylgruppe, die 3-Hydroxypropylgruppe, die 2-Hydroxygpropylgruppe genannt werden.

Die zur anderen Aminogruppe benachbarte CH-Gruppe trägt den Rest R2. Der Rest R2 kann unabhängig vom Rest R1 gewählt werden und kann für ein Wasserstoffatom, eine C₁-C₆-Alkylgruppe oder eine Hydroxy-C₁-C₆-alkylgruppe stehen.

Bevorzugt stehen die Reste R1 und R2 unabhängig voneinander für ein Wasserstoffatom, eine C₁-C₆-Alkylgruppe oder eine Hydroxy-C₁-C₆-alkylgruppe.

Weiterhin können die Reste R1 und R2 auch zusammen mit den beiden Kohlenstoffatomen, an die sie gebunden sind, und dem Linker L einen 5-, 6- oder 7-gliedrigen, gesättigten, carbozyklischen oder heterozyklischen Ring bilden.

Beispiele für carbocyclische, gesättigte Ringe sind ein Cyclopentylring, ein Cyclohexylring und ein Cycloheptylring. Beispiele für heterozyklische, gesättigte Ringe sind ein Pyrrolidinyl-Ring, ein Piperidinyl-Ring und ein Azepan-Ring.

Die Beschleunigung der Kupplungsreaktion der selbstoxidierenden Farbstoffe (SOF) funktionierte besonders gut, wenn Di- oder Polyaminoverbindungen (DA) der Formel (I) eingesetzt wurden, bei denen die Reste R1 und R2 unabhängig voneinander für ein Wasserstoffatom oder eine Methylgruppe stehen. Ganz besonders bevorzugt stehen die Reste R1 und R2 beide für ein Wasserstoffatom.

In einer weiteren ganz besonders bevorzugten Ausführungsform ist eine erfindungsgemäße Mehrkomponenten-Verpackungseinheit dadurch gekennzeichnet, dass die Zusammensetzung (B) mindestens eine aliphatische Di- oder Polyaminoverbindung (DA) der Formel (I) enthält, wobei
- R1 und R2: unabhängig voneinander für ein Wasserstoffatom oder eine Methylgruppe, besonders bevorzugt beide für ein Wasserstoffatom stehen.

Im Strukturbild links ist der Linker L an die Gruppierung H₂N-CH(R1)- gebunden.

Im Strukturbild rechts ist der Linker L and die Gruppierung -CH(R2)-NH₂ gebunden.

In einer Ausführungsform stellt der Linker eine direkte Bindung dar. Wenn der Linker L für eine direkte Bindung steht, handelt es sich bei der Di- oder Polyaminoverbindung (DA) um eine Verbindung der allgemeinen Formel (la)

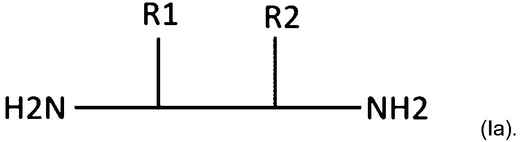

Ein gut geeigneter Vertreter ist zum Beispiel 1,2-Diaminocyclohexan, das alternativ auch als 1,2-Cyclohexandiamin bezeichnet werden kann. 1,2-Diaminocyclohexan enthält zwei stereogene Zentren, so dass hiervon verschiedene Stereoisomere existieren. Alle Stereoisomere sind erfindungsgemäß. Das Isomerengemisch besitzt die CAS-Nummer 694-83-7. Das trans-Isomer besitzt die CAS-Nummer 1121-22-8. Das cis-Isomer besitzt die CAS-Nummer 1436-59-5. Das (1R,2R)-(-)-Enantiomer besitzt die CAS-Nummer 20439-47-8. Das [(1*S*,2*S*)-(+)-Enantiomer] besitzt die CAS-Nummer 21436-03-3.

1,2-Diaminocyclohexan bilden die Reste R1 und R2 zusammen mit den Kohlenstoffatomen, an die sie gebunden sind, einen 6-gliedrigen carbozyklischen gesättigten Ring aus. Der Linker L steht für eine direkte Bindung.

In einer weiteren Ausführungsform steht der Linker L für eine gesättigte oder ungesättigte, lineare oder verzweigte C₁-C₁₂-Alkylengruppe, die gegebenenfalls auch mit einer oder mehreren Hydroxygruppen substituiert und/oder durch eine oder mehrere Sauerstoffatome und/oder durch eine oder mehrere Gruppierungen -NR3- unterbrochen sein kann.

Beispiele für eine lineare C₁-C₁₂-Alkylengruppe sind die Methylengruppe (-CH₂-), die Ethylengruppe (-CH₂-CH₂-), die Propylengruppe (-CH₂-CH₂-CH₂-), die Butylengruppe (-CH₂-CH₂-CH₂-CH₂-), die Pentylengruppe (-CH₂-CH₂-CH₂-CH₂-CH₂-) oder die Hexylengruppe (-CH₂-CH₂-CH₂-CH₂-CH₂-CH₂-).

Beispiele für eine gut geeignete verzweigte C1-C12-Alkylengruppe sind die 1,1-Dimethylmethylengruppe -C(CH₃)₂-, die 1-Methyl-ethylen-gruppe (-CH(CH₃)-CH₂-), die 2-Methylethylengruppe (-CH₂-CH(CH₃)-), die 1-Methylpropylengruppe (-CH(CH₃)-CH₂-CH₂-), die 2-Methylpropylengruppe (-CH2-CH(CH3)-CH2-), die 3-Propylmethylen-gruppe (-CH2-CH2-CH(CH3)-), die 1,1-Dimethylethylengruppe (-C(CH₃)₂-CH₂), die 1,2-Dimethyl-ethylengruppe (-CH(CH₃)-CH(CH₃)-), die 2,2-Dimethyl-ethylengruppe (-CH₂-C(CH₃)₂-), die 1,1-Dimethylpropylengruppe (-C(CH₃)-CH₂-CH₂-), die 1,2-Dimethylpopylengruppe (-CH(CH₃)-CH(CH₃)-CH₂-), die 1,3-Dimethylpropylengruppe (-CH(CH₃)-CH₂-CH(CH₃)-), die 2,2-Dimethylpropylengruppe (-CH₂-C(CH₃)₂-CH₂-), die 1-Ethylethylengruppe (-CH(CH₂CH₃)-CH₂-), die 2-Ethylethylengruppe (-CH₂-CH(CH₂CH₃)-), die 1-Ethylpropylengruppe, (-CH(CH₂CH₃)-CH₂-CH₂-), die 2-Ethylpropylengruppe (-CH₂-CH(CH₂CH₃)-CH₂-) und die 3-Ethylpropylengruppe (-CH₂-CH₂-CH(CH₂CH₃)-).

Die gesättigte oder ungesättigte, lineare oder verzweigte C₁-C₁₂-Alkylengruppe kann auch eine oder mehrere Hydroxygruppen tragen. Beispielhaft zu nennen sind hier die Gruppierungen -CH(OH)-, - CH(OH)-CH2-, -CH2-CH(OH)-, -CH₂-CH(OH)-CH₂-, -CH(OH)-CH(OH)-, -CH2-CH(OH)-CH2- und - CH(OH)-CH(OH)-CH(OH).

Der Linker L kann auch für eine gesättigte oder ungesättigte, lineare oder verzweigte C₁-C₁₂-Alkylengruppe stehen, die durch eine oder mehrere Sauerstoffatome unterbrochen ist. Beispielhaft zu nennen ist hier eine Gruppierung -CH₂-O-CH₂-, eine Gruppierung -CH₂-O-CH₂-CH₂-O-CH₂-, eine Gruppierung -CH₂-O-CH₂-CH₂-O-CH₂-CH₂-O-CH₂- und eine Gruppierung -CH2-CH2-O-CH2-CH2-.

Der Linker L kann auch für eine gesättigte oder ungesättigte, lineare oder verzweigte C₁-C₁₂-Alkylengruppe stehen, die durch eine oder mehrere Gruppierungen -NR3- unterbrochen ist. Der Rest R3 steht hierbei für ein Wasserstoffatom, eine C₁-C₆-Alkylgruppe, eine Hydroxy-C₁-C₆-alkylgruppe oder eine Amino-C₁-C₆-Alkylgruppe. Beispiele für gut geeignete C₁-C₆-Alkylgruppen und Hydroxy-C₁-C₆-alkylgruppen wurden bereits zuvor genannt.

Beispiele für gut geeignete Amino-C₁-C₆-Alkylgruppen sind die 2-Aminoethylgruppe, die 3-Aminopropylgruppe, die 2-Aminopropylgruppe und die 4-Aminobutylgruppe. Die 2-Aminoethylgruppe und die 3-Aminopropylgruppe sind ganz besonders bevorzugt.

Besonders bevorzugt steht der Rest R3 für ein Wasserstoffatom, eine Methylgruppe, eine 2-Aminoethylgruppe oder eine 3-Aminopropylgruppe.

Beispielhaft zu nennen ist hier eine Gruppierung -CH₂-NH-CH₂-, eine Gruppierung -CH₂-NH-CH₂-CH₂-NH-CH₂-, eine Gruppierung -CH₂-NH-CH₂-CH₂-NH-CH₂-CH₂-NH-CH₂, eine Gruppierung -CH₂-NH-CH₂-CH₂-NH-CH₂-CH₂-NH-CH₂- , eine Gruppierung -CH₂-CH₂-NR3-CH₂-CH₂- und eine Gruppierung -CH₂-CH₂-CH₂-NR3-CH₂-CH₂-CH₂-.

In einer weiteren ganz besonders bevorzugten Ausführungsform ist ein erfindungsgemäße Mehrkomponenten-Verpackungseinheit dadurch gekennzeichnet, dass die Zusammensetzung (B) mindestens eine aliphatische Di- oder Polyaminoverbindung (DA) der Formel (I) und/oder deren Salz enthält, wobei
- L: für eine gesättigte, lineare C₁-C₁₂-Alkylengruppe, eine gesättigte, verzweigte C₃-C₁₂-Alkylengruppe, eine Gruppierung -CH(OH)-, eine Gruppierung -CH₂-CH(OH)-CH₂-, eine Gruppierung -CH₂-O-CH₂-, eine Gruppierung -CH₂-O-CH₂-CH₂-O-CH₂-, eine Gruppierung -CH₂-O-CH₂-CH₂-O-CH₂-CH₂-O-CH₂-, eine Gruppierung -CH2-CH2-O-CH2-CH2-, eine Gruppierung -CH₂-NH-CH₂-, eine Gruppierung -CH₂-NH-CH₂-CH₂-NH-CH₂-, eine Gruppierung - CH₂-NH-CH₂-CH₂-NH-CH₂-CH₂-NH-CH₂-, eine Gruppierung -CH₂-NH-CH₂-CH₂-NH-CH₂-CH₂-NH-CH₂-, eine Gruppierung -CH₂-CH₂-NR3-CH₂-CH₂-oder eine Gruppierung -CH₂-CH₂-CH₂-NR3-CH₂-CH₂-CH₂- steht.

In einer weiteren bevorzugten Ausführungsform ist eine erfindungsgemäße Mehrkomponenten-Verpackungseinheit auch dadurch gekennzeichnet, dass mindestens eine aliphatische Di- oder Polyaminoverbindung (DA) der Formel (I) und/oder deren Salz auf den keratinischen Fasern angewendet wird, wobei
- L: eine Gruppierung -CH₂-NR3-CH₂- oder eine Gruppierung -CH₂-CH₂-NR3-CH₂-CH₂- steht, und
- R3: für eine 2-Aminoethylgruppe oder eine 3-Aminopropylgruppe steht.

Als ganz besonders gut geeignet zur Lösung der erfindungsgemäßen Aufgabenstellung haben sich die aliphatischen Di- oder Polyaminoverbindungen der Formel (I) erwiesen, die ausgewählt sind aus der Gruppe aus 1,6-Diaminohexan, 1,5-Diaminopentan, 1,4-Diaminobutan, 1,3-Diamino-propan, 1,8-Diamino-octan, 1,10-Diamino-decan, 1,12-Diamino-dodecan, 1,5-Diamino-2-methyl-pentan, 1,3-Diamino-pentan, 1,3-Diamino-2,2-dimethylpropan,1,3-Diamino-2-propanol, 2-(2-Aminoethoxy)ethylamin, 2,2'-(Ethylendioxy)diethylamin, Bis(3-Aminopropyl)ether, Diethylentriamin, Triethylentetramin, Tetraethylenpentamin, Tris(3-aminopropyl)amin, Tris(2-aminoethyl)amin und den Salzen dieser Verbindungen.

### 1,6-Diaminohexan besitzt die CAS-Nummer 124-09-4.

Bei 1,6-Diaminohexan stehen die Reste R1 und R2 beide für ein Wasserstoffatom, und der Linker L steht für eine Butylengruppe (-CH₂-CH₂-CH₂-CH₂-). 1,6-Diaminohexan ist am allermeisten bevorzugt

### 1,5-Diaminopentan besitzt die CAS-Nummer 462-94-2.

Bei 1,5-Diaminopentan stehen die Reste R1 und R2 beide für ein Wasserstoffatom, und der Linker L steht für eine Propylengruppe (-CH₂-CH₂-CH₂-).

### 1,4-Diaminobutan besitzt die CAS-Nummer 110-60-1.

Bei 1,4-Diaminobutan stehen die Reste R1 und R2 beide für ein Wasserstoffatom, und der Linker L steht für eine Ethylengruppe (-CH₂-CH₂-).

### 1,3-Diaminopropan besitzt die CAS-Nummer 109-76-2.

Bei 1,3-Diaminopropan stehen die Reste R1 und R2 beide für ein Wasserstoffatom, und der Linker L steht für eine Methylengruppe (-CH₂-).

### 1,8-Diaminooctan trägt die CAS-Nummer 373-44-4.

Bei 1,3-Diaminopropan stehen die Reste R1 und R2 beide für ein Wasserstoffatom, und der Linker L steht für eine Hexamethylengruppe.

### 1,10-Diaminodecan trägt die CAS-Nummer 646-25-3.

Bei 1,3-Diaminopropan stehen die Reste R1 und R2 beide für ein Wasserstoffatom, und der Linker L steht für eine Octamethylengruppe.

### 1,12-Diamino-dodecan trägt die CAS-Nummer 2783-17-7.

Bei 1,12-Diaminododecan stehen die Reste R1 und R2 beide für ein Wasserstoffatom, und der Linker L steht für eine Decamethylengruppe.

### 1,5-Diamino-2-methyl-pentan trägt die CAS-Nummer 15520-10-2.

Bei 1,5-Diamino-2-methyl-pentan stehen die Reste R1 und R2 beide für ein Wasserstoffatom, und der Linker L steht für die verzweigte Alkylengruppe -CH(CH₃)-CH₂-CH₂-.

### 1,3-Diamino-pentan besitzt die CAS-Nummer 589-37-7.

1,3-Diamino-pentan steht der Rest R1 für eine Ethylgruppe und der Rest R2 für ein Wasserstoffatom. Der Linker L steht für eine Methylengruppe (-CH₂-).

### 1,3-Diamino-2,2-dimethylpropan trägt die CAS-Nummer 7328-91-8.

Bei 1,3-Diamino-2,2-dimethylpropan stehenj die Reste R1 und R2 beide für ein Wasserstoffatom, und der Linker L steht für die verzweigte Alkylengruppe -C(CH₃)₂-.

### 1,3-Diamino-2-propanol besitzt die CAS-Nummer 616-29-5.

Bei 1,3-Diamino-2-propanol stehen die Reste R1 und R2 beide für ein Wasserstoffatom, und er Linker L steht für eine Gruppierung -CH(OH)-.

### 2-(2-Aminoethoxy)ethylamin besitzt die CAS-Nummer 2752-17-2.

Bei 2-(2-Aminoethoxy)ethylamin stehen die Reste R1 und R2 beide für ein Wasserstoffatom, und der Linker L steht für eine Gruppierung -CH2-O-CH2-. Die Verbindung kann entweder in Substanz eingesetzt werden oder aber in Form ihres Dihydrochlorids.

### 2,2'-(Ethylendioxy)diethylamin besitzt die CAS-Nummer 929-59-9.

Bei - 2,2'-(Ethylendioxy)diethylamin stehen die Reste R1 und R2 beide für ein Wasserstoffatom, und der Linker L steht für eine Gruppierung -CH₂-O-CH₂-CH₂-O-CH₂-.

### Bis(3-Aminopropyl)ether besitzt die CAS-Nummer 2157-24-6.

Bei Bis(3-Aminopropyl)ether stehen beide Reste R1 und R2 für ein Wasserstoffatom, und der Linker L steht für eine Gruppierung -CH₂-CH₂-O-CH₂-CH₂-.

### Diethylentriamin besitzt die CAS-Nummer 111-40-0.

Bei Diethylentriamin stehen beide Reste R1 und R2 für ein Wasserstoffatom, und der Linker L steht für eine Gruppierung -CH₂-HN-CH₂-.

### Triethylentetramin besitzt die CAS-Nummer 112-24-3.

Bei Triethylentetramin stehen die Reste R1 und R2 beide für ein Wasserstoffatom, und der Linker L steht für eine Gruppierung -CH₂-NH-CH₂-CH₂-NH-CH₂-. Triethylentetramin kann auch in Form seines Dihydrochlorids eingesetzt werden, welches die CAS-Nummer 38260-01-4 besitzt.

### Tetraethylenpentamin besitzt die CAS-Nummer 112-57-2.

Bei Tetraethylenpentamin stehen beide Reste R1 und R2 für ein Wasserstoffatom, und der Linker L steht für eine Gruppierung -CH2-NH-CH2-CH2-NH-CH2-CH2-NH-CH2-.

### Tris(3-aminopropyl)amin trägt die CAS-Nummer 4963-47-7.

Tris(3-aminopropyl)amin stehen beide Reste R1 und R2 für ein Wasserstoffatom, und der Linker L steht für eine Gruppierung -CH2-CH2-NR3-CH2-CH2-, wobei der Rest R3 für eine 3-Aminopropylgruppe steht.

### Tris(2-aminoethyl)amin besitzt die CAS-Nummer 4097-89-6.

Bei Tris(2-aminoethyl)amin stehen beide Reste R1 und R2 für ein Wasserstoffatom, und der Linker L steht für eine Gruppierung -CH2-NR3-CH2, wobei der Rest R3 für eine 2-Aminoethylgruppe steht.

### In einer weiteren ganz besonders bevorzugten Ausführungsform ist eine erfindungsgemäße

Mehrkomponenten-Verpackungseinheit dadurch gekennzeichnet, dass die Zusammensetzung (B) mindestens eine die aliphatische Di- oder Polyaminoverbindung der Formel (I) enthält, die ausgewählt ist aus der Gruppe aus 1,6-Diaminohexan, 1,5-Diaminopentan, 1,4-Diaminobutan, 1,3-Diaminopropan, 1,8-Diamino-octan, 1,10-Diamino-decan, 1,12-Diamino-dodecan, 1,5-Diamino-2-methylpentan, 1,3-Diamino-pentan, 1,3-Diamino-2,2-dimethylpropan,1,3-Diamino-2-propanol, 2-(2-Amino-ethoxy)ethylamin, 2,2'-(Ethylendioxy)diethylamin, Bis(3-Aminopropyl)ether, Diethylentriamin, Triethylentetramin, Tetraethylenpentamin, Tris(3-aminopropyl)amin, Tris(2-aminoethyl)amin und deren Salzen.

Eine besonders starke Farbintensivierung ausgehend von 5,6-Dihydroxyindolin wurde mit 1,6-Diaminohexan erhalten. Aus diesem Grund ist eine Mehrkomponenten-Verpackungseinheit, die eine Zusammensetzung (A) mit 5,6-Dihydroxyindolin und eine Zusammensetzung (B) mit 1,6-Diaminohexan (und/oder dessen Salz) umfasst, am allermeisten bevorzugt.

Auch eine Mehrkomponenten-Verpackungseinheit, die eine Zusammensetzung (A) mit 5,6-Dihydroxyindolin Hydrobromid und eine Zusammensetzung (B) mit 1,6-Diaminohexan (und/oder einem Salz hiervon) umfasst, ebenfalls explizit ganz besonders bevorzugt

### Mischung der selbstoxidierenden Farbstoffe (SOF) und der aliphatischen Di- oder Polyamine (DA)

Kurz vor Anwendung auf den keratinischen Fasern werden die Zusammensetzungen (A) und (B) miteinander vermischt, damit das oder die aliphatischen Di- oder Polyamine (DA) mit den selbstoxidierenden Farbstoffen (SOF) in Wechselwirkung treten können. Ein Ergebnis dieser Wechselwirkung ist die Beschleunigung und Verstärkung der luftsauerstoffinduzierten Farbstoffbildungsreaktion.

Die Mischung aus (A) und (B) kann auch als anwendungsbereites Färbemittel oder Anwendungsmischung bezeichnet werden.

Die Intensität des auf den Haaren erhaltenen Farbergebnisses wird hierbei von der Einsatzmenge der selbstoxidierenden Farbstoffe (SOF) und der Menge an eingesetzten aliphatischen Di- oder Polyaminen (DA) mitbestimmt.

Dem Fachmann ist bekannt, dass die Intensität einer entwickelten Farbnuance von der Menge der selbstoxidierenden Farbstoffe abhängt, die er im anwendungsbereiten Mittel einsetzt. Wünscht der Fachmann eine besonders dunkle Nuance, wird er die Mengen an (SOF) entsprechend erhöhen.

Es hat sich aber herausgestellt, dass sich die Farbintensität nur bis zu einem bestimmten Grenzwert proportional zur Einsatzmenge der (SOF) verhält. Die Versuche haben gezeigt, dass die auf den Keratinfasern erhaltene Nuance dunkler wurde, wenn die Einsatzmenge der (SOF) von 0,5 Gew.-% auf 1,0 Gew.-% (bezogen auf das Gesamtgewicht des anwendungsbereiten Mittels) erhöht wurde. Bei einer Menge von 1,5 Gew.-% waren die Keratinfasern aber bereits so dunkel gefärbt, dass eine weitere starke Erhöhung der Menge an (SOF) keine weitere signifikante Erhöhung der Farbintensität mehr brachte. Aus diesem Grund ist es bevorzugt, wenn das anwendungsbereite Färbemittel - bezogen auf das Gesamtgewicht des anwendungsbereiten Färbemittels - einen oder mehrere Oxidationsfarbstoffvorprodukte, insbesondere einen oder mehrere selbstoxidierende Farbstoffe (SOF), in einer Gesamtmenge von 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% enthält.

In einer weiteren ganz besonders bevorzugten Ausführungsform ist eine erfindungsgemäße Mehrkomponenten-Verpackungseinheit dadurch gekennzeichnet, dass die Mischung der Zusammensetzungen (A) und (B) - bezogen auf das Gesamtgewicht der Mischung der Zusammensetzungen (A) und (B) - einen oder mehrere Oxidationsfarbstoffvorprodukte, besonders bevorzugt einen oder mehrere selbstoxidierende Farbstoffe (SOF), in einer Gesamtmenge von 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% enthält.

In einer weiteren explizit ganz besonders bevorzugten Ausführungsform ist eine erfindungsgemäße Mehrkomponenten-Verpackungseinheit dadurch gekennzeichnet, dass die Mischung der Zusammensetzungen (A) und (B) - bezogen auf das Gesamtgewicht der Mischung der Zusammensetzungen (A) und (B) - 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% 5,6-Dihydroxyindolin, Hydrobromid enthält.

Auch die Menge der im anwendungsbereiten Färbemittel eingesetzten aliphatischen Di- oder Polyaminen (DA) nimmt auf die Intensität der Färbung einen Einfluss. Besonders gute Ergebnisse wurden erhalten, wenn das anwendungsbereite Färbemittel - bezogen auf sein Gesamtgewicht - eine oder mehrere aliphatische Di- oder Polyaminoverbindungen (DA) der Formel (I) in einer Gesamtmenge von Gesamtmenge von 0,01 bis 20,0 Gew.-%, bevorzugt von 0,1 bis 10,0 Gew.-%, weiter bevorzugt 0,2 bis 5,0 Gew.-% noch weiter besonders bevorzugt 0,3 bis 2,5 Gew.-% und ganz besonders bevorzugt von 0,5 bis 1,0 Gew.-% enthielt.

In einer weiteren ganz besonders bevorzugten Ausführungsform ist eine erfindungsgemäße Mehrkomponenten-Verpackungseinheit dadurch gekennzeichnet, dass die Mischung der Zusammensetzungen (A) und (B) - bezogen auf das Gesamtgewicht der Mischung der Zusammensetzungen (A) und (B) - eine oder mehrere aliphatische Di- oder Polyaminoverbindungen (DA) der Formel (I) in einer Gesamtmenge von Gesamtmenge von 0,01 bis 20,0 Gew.-%, bevorzugt von 0,1 bis 10,0 Gew.-%, weiter bevorzugt 0,2 bis 5,0 Gew.-% noch weiter besonders bevorzugt 0,3 bis 2,5 Gew.-% und ganz besonders bevorzugt von 0,5 bis 1,0 Gew.-% enthält.

In einer weiteren ganz besonders bevorzugten Ausführungsform ist eine erfindungsgemäße Mehrkomponenten-Verpackungseinheit dadurch gekennzeichnet, dass die Mischung der Zusammensetzungen (A) und (B) - bezogen auf das Gesamtgewicht der Mischung der Zusammensetzungen (A) und (B) - 0,01 bis 20,0 Gew.-%, bevorzugt von 0,1 bis 10,0 Gew.-%, weiter bevorzugt 0,2 bis 5,0 Gew.-% noch weiter besonders bevorzugt 0,3 bis 2,5 Gew.-% und ganz besonders bevorzugt von 0,5 bis 1,0 Gew.-% 1,6-Diaminohexan enthält.

Bei den zu dieser Erfindung führenden Versuchen hat sich weiterhin gezeigt, dass das Ausmaß und die Schnelligkeit der Farbstoffbildung auch von dem Gewichtsverhältnis abhängen, in dem der oder die selbstoxidierenden Farbstoffe (SOF) und das oder die aliphatischen Di- bzw. Polyamine (DA) zueinander eingesetzt werden.

Eine sehr gute Verstärkung der Farbstoffentwicklung wurde bereits erhalten, wenn das Gewichtsverhältnis der auf den Fasern angewendeten selbstoxidierenden Farbstoffe (SOF) zu den Di- oder Polyaminen (DA) der Formel (I), d.h. das Gewichtsverhältnis (SOF)/(DA), bei einem Bereich von 4:1 bis 1:4 lag. Mit weiterer Anpassung des Gewichtsverhältnisses konnte die Farbintensität aber noch weiter erhöht werden. Aus diesem Grund ist es ganz besonders bevorzugt, wenn der oder die selbstoxidierenden Farbstoffe (SOF) und das oder die Di- oder Polyamine (DA) der Formel (I) in einem Gewichtsverhältnis (SOF)/(DA) im Bereich von 4:1 bis 1:4, bevorzugt von 3:1 bis 1:1, weiter bevorzugt von 2,5:1 bis 1:1 und ganz besonders bevorzugt von 2,5:1 bis 1,5:1 auf die Fasern appliziert werden.

In einer weiteren ganz besonders bevorzugten Ausführungsform ist eine erfindungsgemäße Mehrkomponenten-Verpackungseinheit dadurch gekennzeichnet, dass das in der Mischung der Zusammensetzungen (A) und (B) vorhandene Gewichtsverhältnis aus allen selbstoxidierenden Farbstoffen (SOF) zu allen Di- oder Polyaminen (DA) der Formel (I), d.h. das Gewichtsverhältnis (SOF)/(DA), im Bereich von 4:1 bis 1:4, bevorzugt von 3:1 bis 1:1, weiter bevorzugt von 2,5:1 bis 1:1 und ganz besonders bevorzugt von 2,5:1 bis 1,5:1 liegt.

Bei dem ganz besonders bevorzugten Gewichtsverhältnis (SOF)/(DA) von 2,5:1 bis 1,5:1 werden das oder die selbstoxidierenden Farbstoffe (SOF) im Vergleich zu dem oder den zu Diaminen (DA) in einem 1,5-fachen bis 2,5-fachen Gewichtsüberschuss auf den keratinischen Fasern angewendet.

In einer weiteren explizit ganz besonders bevorzugten Ausführungsform ist eine erfindungsgemäße Mehrkomponenten-Verpackungseinheit dadurch gekennzeichnet, dass das in der Mischung der Zusammensetzungen (A) und (B) vorhandene Gewichtsverhältnis von 5,6-Dihydroxyindolin, Hydrobromid zu 1,6-Diaminohexan im Bereich von 4:1 bis 1:4, bevorzugt von 3:1 bis 1:1, weiter bevorzugt von 2,5:1 bis 1:1 und ganz besonders bevorzugt von 2,5:1 bis 1,5:1 liegt.

### pH-Wert des anwendungsbereiten Färbemittels

Aufgrund des neutral bis sauer eingestellten pH-Werts der Zusammensetzung (A) besitzt auch die aus den Zusammensetzungen (A) und (B) hergestellte Mischung einen entsprechend niedrigen pH-Wert. Aufgrund des geringen Misch-pH-Wertes kann eine durch Alkalisierungsmittel induzierte Haarschädigung vermieden werden. Besonders gute Ergebnisse wurden erhalten, wenn die Mischung der Zusammensetzungen (A) und (B) einen pH-Wert von 4,3 bis 9,8, bevorzugt von 4,6 bis 9,0, weiter bevorzugt von 4,8 bis 8,0 und ganz besonders bevorzugt von 5,0 bis 7,5 besaß.

In einer weiteren ganz besonders bevorzugten Ausführungsform ist eine erfindungsgemäße Mehrkomponenten-Verpackungseinheit dadurch gekennzeichnet, dass die Mischung der Zusammensetzungen (A) und (B) einen pH-Wert von 4,3 bis 9,8, bevorzugt von 4,6 bis 9,0, weiter bevorzugt von 4,8 bis 8,0 und ganz besonders bevorzugt von 5,0 bis 7,5 besitzt.

### Verzicht auf von Luftsauerstoff verschiedene Oxidationsmittel und aromatische Diaminoverbindungen

Wie bereits beschrieben, wird unter der durch Luftsauerstoff induzierten oxidativen Färbung verstanden, dass der in der Luft enthaltene Sauerstoff als einziges Oxidationsmittel zur Anwendung kommt. Im erfindungsgemäßen Verfahren werden daher keine Oxidationsmittel wie Wasserstoffperoxid und Peroxodisulfate angewendet.

Bevorzugt ist die erfindungsgemäße Mehrkomponenten-Verpackungseinheit dadurch gekennzeichnet, dass sie keine Zusammensetzung umfasst, die eine oder mehrere Peroxoverbindungen enthält.

Hiermit ist gemeint, dass bevorzugt weder die Zusammensetzung (A) noch die Zusammensetzung (B) eine Peroxoverbindung beinhaltet, d.h. der Gehalt an Peroxoverbindungen in der Zusammensetzung (A) und in der Zusammensetzung (B) liegt jeweils bevorzugt bei 0 Gew.-%, bezogen auf das Gesamtgewicht der jeweiligen Zusammensetzung.

Auch wenn die Mehrkomponenten-Verpackungseinheit optional noch eine weitere Zusammensetzung (C) oder mehrere weitere Zusammensetzungen (C) plus (D) umfasst, sind diese bevorzugt frei von Peroxoverbindungen.

Unter dem Begriff Peroxoverbindungen werden erfindungsgemäß anorganische und organische Verbindungen verstanden, die durch die Anwesenheit einer Peroxygruppe -O-O- gekennzeichnet sind. Hierbei besitzen die Sauerstoffatome in diesen Peroxoverbindungen in der Regel die Oxidationsstufe -1.

Bei Peroxoverbindungen im Sinne der Erfindung handelt es sich demnach um die Verbindungen der allgemeinen Formel (O-1)

Ro₁-O-O-Ro₂ (O-1)

wobei die Reste Ro1 und Ro2 für ein Wasserstoffatom, für einen anorganischen Rest oder für einen organischen Rest stehen.

Peroxoverbindungen sind Oxidationsmittel, die üblicherweise in der oxidativen Haarfärbung eingesetzt werden, um die Reaktion zwischen Entwickler und Kuppler zu initiieren, die zur Ausbildung der Farbstoffe führt.

Bei der am häufigsten in der oxidativen Haarfärbung eingesetzten Peroxoverbindung handelt es sich um Wasserstoffperoxid (mit Ro1 und Ro2 gleich Wasserstoff).

Bei ebenfalls sehr häufig in der oxidativen Haarfärbung eingesetzten Peroxoverbindungen handelt es sich um Kaliumpersulfat (auch als Kaliumperoxodisulfat bezeichnet), Natriumpersulfat (auch als Natriumperoxodisulfat bezeichnet) und Ammoniumpersulfat (auch als Ammoniumperoxodisulfat bezeichnet). Im Fall der Persulfate stellen die Reste Ro1 und Ro2 jeweils einen Rest -SO3- dar, wobei die negative Ladung jeweils durch die Anwesenheit der entsprechenden Kationen neutralisiert wird.

Bei einer ebenfalls in einigen oxidativen Haarfärbemitteln eingesetzten Peroxoverbindung handelt es sich um Percarbonate wie Natriumpercarbonat, Kaliumpercarbonat und Magnesiumpercarbonat.

Percarbonate sind Anlagerungsprodukte (Addukte) von Wasserstoffperoxid an die entsprechenden Carbonate, beispielsweise ist Natriumpercarbonat das Anlagerungsprodukt von Wasserstoffperoxid an Natriumcarbonat. Damit stellen auch die Percarbonate Peroxoverbindungen war, wobei den Percarbonaten als Peroxoverbindungen das Wasserstoffperoxid zu Grunde liegt.

Im Rahmen einer weiteren bevorzugten Ausführungsform ist eine erfindungsgemäße Mehrkomponenten-Verpackungseinheit daher dadurch gekennzeichnet, dass
- die Zusammensetzung (A) kein Oxidationsmittel aus der Gruppe aus Wasserstoffperoxid, den Anlagerungsverbindungen von Wasserstoffperoxid an organische Verbindungen den Anlagerungsverbindungen von Wasserstoffperoxid an anorganische Verbindungen, Kaliumpersulfat, Natriumpersulfat und Ammoniumpersulfat enthält, und
- die Zusammensetzung (B) kein Oxidationsmittel aus der Gruppe aus Wasserstoffperoxid, den Anlagerungsverbindungen von Wasserstoffperoxid an organische Verbindungen den Anlagerungsverbindungen von Wasserstoffperoxid an anorganische Verbindungen, Kaliumpersulfat, Natriumpersulfat und Ammoniumpersulfat, enthält.

### Verzicht auf weitere Alkalisierungsmittel

Die mit den erfindungsgemäßen Anwendungsmischungen (A) + (B) erhaltenen Färbungen liefern intensive Farbergebnisse bei niedrigen pH-Werten. Wie bereits beschrieben besitzt das anwendungsbereite Färbemittel einen pH-Wert, der bevorzugt im leicht sauren bis neutralen Bereich von 4,8 bis 8,0 und ganz besonders bevorzugt im Bereich von 5,0 bis 7,5 liegt.

Aufgrund dieser pH-Werte kann auf die Alkalisierungsmittel, die für die aus dem Stand der Technik bekannten Färbemittel bislang obligatorisch waren, verzichtet werden.

Üblicherweise in der Haarfärbung eingesetzte Alkalisierungsmittel sind beispielsweise Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin.

In einer weiteren ganz besonders bevorzugten Ausführungsform ist eine erfindungsgemäße Mehrkomponenten-Verpackungseinheit dadurch gekennzeichnet, dass sie keine Zusammensetzung umfasst, die ein oder mehrere Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält.

Hiermit ist gemeint, dass bevorzugt weder die Zusammensetzung (A) noch die Zusammensetzung (B) ein Alkalisierungsmittel aus der vorgenannten Gruppe beinhalten, d.h. der Gehalt an diesen Alkalisierungsmitteln in der Zusammensetzung (A) und in der Zusammensetzung (B) liegt jeweils bevorzugt bei 0 Gew.-%, bezogen auf das Gesamtgewicht der jeweiligen Zusammensetzung.

In einer weiteren ganz besonders bevorzugten Ausführungsform ist eine erfindungsgemäße Mehrkomponenten-Verpackungseinheit dadurch gekennzeichnet, dass
- die Zusammensetzung (A) keine Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält, und
- die Zusammensetzung (B) keine Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält.

Wenn die Mehrkomponenten-Verpackungseinheit optional noch eine weitere Zusammen-setzung (C) oder mehrere weitere Zusammensetzungen (C) plus (D) umfasst, sind diese bevorzugt frei von Alkalisierungsmitteln aus der vorgenannten Gruppe.

Die Zusammensetzungen (A) und (B) können in verschiedenen Mengenverhältnissen miteinander vermischt werden. So können (A) und (B) beispielsweise in einem Mengenverhältnis von 1:10 bis 10:1, bevorzugt 1:5 bis 5:1, weiter bevorzugt 1:3 bis 3:1 und besonders bevorzugt 1:2 bis 2:1 miteinander vermischt werden. Diese Mischungsverhältnisse sind insbesondere dann von Vorteil, wenn die Oxidationsfarbstoffvorprodukte, bzw. die selbstoxidierenden Farbstoffe (SOF), in der Zusammensetzung (A) und die Di- oder Polyaminen (DA) in der Zusammensetzung (B) in vergleichbaren und verhältnismäßig geringen Konzentrationen verwendet werden.

Es kann aber auch sein, dass die selbstoxidierenden Farbstoffe (SOF), in der Zusammensetzung (A) oder die Di- oder Polyaminen (DA) in der Zusammensetzung (B) verhältnismäßig konzentriert eingesetzt werden. In Rahmen einer solchen Ausführungsform ist es auch bevorzugt, wenn (A) und (B) in einem Mengenverhältnis von 150:1 bis 1:150 miteinander vermischt werden.

Wenn beispielsweise 99,3 Gramm der Zusammensetzung (A) mit 0,7 Gramm des Diamins (DA) (welches als Rohstoff an sich eingesetzt wird, so dass die Zusammensetzung (B) im Prinzip aus dem Diamin besteht), dann liegt das Mischungsverhältnis (bezogen auf das Gewicht der beiden Zusammensetzungen) bei 142:1.

### Weiter optionale Bestandteile in den Mitteln (A) und (B) und/oder im anwendungsbereiten Färbemittel

Die Zusammensetzungen (A) und/oder (B) und/oder das anwendungsbereite Färbemittel können optional auch noch weitere Wirk-, Hilfs- und Zusatzstoffe enthalten, wie beispielsweise nichtionische, kationische, anionische, zwitterionische und/oder amphotere Tenside, Fettstoffe wie beispielsweise Fettalkohole, Fettsäuretriglyceride oder Kohlenwasserstoffe, nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Zusammensetzung (B) kann optional auch einen oder mehrere direktziehende Farbstoffe enthalten.

Bevorzugt enthält Zusammensetzung (B) zusätzlich mindestens einen direktziehenden Farbstoff aus der Gruppe der kationischen, anionischen und/oder nichtionischen direktziehenden Farbstoffe.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beispielsweise nichtionische Nitro- und Chinonfarbstoffe und neutrale Azofarbstoffe genannt werden. Beispiele für nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Als Beispiele für Säurefarbstoffe können genannt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI 27290), Acid Red 87 (Eosin, CI 45380), Acid Red 92 (COLIPA n° C53, CI 45410), Acid Red 95 (CI 45425, Erythtosine, Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

### anwendungsbereites Färbemittel

Wie bereits zuvor beschrieben wird kurz vor der Anwendung durch Vermischen der beiden Zusammensetzungen (A) und (B) ein anwendungsbereites Färbemittel hergestellt, welches dann auf die keratinischen Fasern aufgetragen wird.

Ein zweiter Gegenstand der vorliegenden Anmeldung ist daher ein anwendungsbereites Färbemittel, das hergestellt wird durch Vermischen von
- einer Zusammensetzung (A), wie sie bei der Beschreibung des ersten Erfindungsgegenstand bereits im Detail offenbart wurde, und
- einer Zusammensetzung (B), bei der Beschreibung des ersten Erfindungsgegenstand im Detail offenbart wurde.

Betreffend die weiteren bevorzugten Ausführungsformen des erfindungsgemäßen anwendungsbereiten Färbemittels gilt *mutatis mutantis* das zur erfindungsgemäßen Mehrkomponenten-Verpackungseinheit gesagte.

### Verfahren zur durch Luftsauerstoff induzierten Färbung

Bei dem erfindungsgemäßen Verfahren zur durch Luftsauerstoff induzierten Färbung keratinischer Fasern, insbesondere menschlicher Haare, werden der oder die selbstoxidierenden Farbstoffe (SOF) und eine oder mehrere aliphatische Di- oder Polyaminoverbindung (DA) der Formel (I) und/oder deren Salze auf den keratinischen Fasern angewendet.

Unter einer Färbung, die durch Luftsauerstoff induziert ist, wird im Sinne der vorliegenden Anmeldung verstanden, dass der in der Luft enthaltene Sauerstoff als einziges Oxidationsmittel zur Anwendung kommt.

Ein dritter Gegenstand der vorliegenden Anmeldung ist daher ein Verfahren zur durch Luftsauerstoff induzierten Färbung keratinhaltiger Fasern, insbesondere menschlicher Haare, umfassend die folgenden Schritte:
- Bereitstellen einer Zusammensetzung (A), wie sie bei der Beschreibung des ersten Erfindungsgegenstand bereits im Detail offenbart wurde,
- Bereitstellen einer Zusammensetzung (B), wie sie bei der Beschreibung des ersten Erfindungsgegenstand bereits im Detail offenbart wurde,
- Herstellung eines anwendungsbereiten Färbemittels durch Vermischen der Zusammensetzungen (A) und (B),
- Auftragen der Anwendungsmischung aus (A) und (B) auf die keratinischen Fasern,
- Einwirken der auf die keratinischen Fasern aufgetragenen Mischung aus (A) und (B) auf die Fasern, und
- gegebenenfalls Ausspülen der Mischung aus (A) und (B) aus den Fasern.

Das Bereitstellen der Zusammensetzungen (A) und (B) kann beispielsweise in Form des zuvor detailliert beschriebenen Kit-of-parts erfolgen. Ein Friseur wendet üblicherweise keinen Kit-of-parts an, sondern arbeitet mit Zusammensetzungen (A) und (B) in größeren Gebinden, die er getrennt voneinander käuflich erwerben kann. Auch diese Art der Bereitstellung ist erfindungsgemäß.

Die Herstellung eines anwendungsbereiten Färbemittels durch Vermischen der Zusammensetzungen (A) und (B) kann beispielsweise erfolgen, indem die Zusammensetzung (A) ein einem für die Anwendung geeigneten Container vorgelegt und die Zusammensetzung (B) zudosiert wird. Es kann aber auch die Zusammensetzung (B) vorgelegt werden und kurz vor der Anwendung die Zusammensetzung (A) hinzugegeben werden. Bei Bereitstellung in größeren Gebinden können beispielsweise auch beide Zusammensetzungen (A) und (B) in ein Mischgefäß dosiert werden.

Bei dem Vermischen kann es sich beispielsweise um ein Verrühren oder ein Verschütteln oder eine andere Methode handeln, die gewährleistet, dass beide Zusammensetzungen in eine homogene Anwendungsmischung überführt werden.

Das Auftragen der Anwendungsmischung aus (A) und (B) auf die keratinischen Fasern bzw. Haaren kann beispielsweise mit der behandschuhten Hand, einer Aplizette oder einem Pinsel erfolgen.

Nach dem Auftragen wird die Mischung aus (A) und (B) auf die keratinische Fasern einwirken gelassen, typische Einwirkzeiten sind beispielsweise Zeiträume von 5 bis 45 Minuten, bevorzugt 10 bis 30 Minuten.

Nach dem Einwirken wird das Färbemittel dann bevorzugt wieder aus den Fasern ausgewaschen, wobei das Auswaschen entweder nur mit Wasser oder mit Wasser unter Zuhilfenahme eines Shampoos erfolgen kann.

Im erfindungsgemäßen Verfahren müssen keine Oxidationsmittel wie Wasserstoffperoxid und Peroxodisulfate angewendet werden, und wie bereits zuvor beschrieben kann auch auf die üblichen Alkalisierungsmittel verzichtet werden.

In einer bevorzugten Ausführungsform ist das erfindungsgemäße Verfahren daher dadurch gekennzeichnet, dass im Verfahren
- keine Zusammensetzung auf den keratinischen Fasern angewendet wird, die eine oder mehrere Peroxoverbindungen enthält, und/oder
- keine Zusammensetzung auf den keratinischen Fasern angewendet wird, die ein Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält.

Betreffend die weiteren bevorzugten Ausführungsformen des erfindungsgemäßen Verfahrens gilt *mutatis mutantis* das zur erfindungsgemäßen Mehrkomponenten-Verpackungseinheit gesagte.

### Beispiele

### 1. Herstellung der Zusammensetzungen (A) und (B)

Es wurden die folgenden Zusammensetzungen (A) und (B) hergestellt:

**Tabelle 1**

| Zusammensetzung (A) | (A1) (Gew.-%) | (A2) (Gew.-%) | (A3) (Gew.-%) | (A4) (Gew.-%) |
|---|---|---|---|---|
| 5,6-Dihydroxyindolin, Hydrobromid (SOF) | 1,50 g | 1,50 g | 1,50 g | 1,50 g |
| Ascorbinsäure | ad pH 3,5 | ad pH 5,4 | ad pH 7,8 | ad pH 9,8 |
| Wasser (dest.) | ad 99,30 g | ad 99,30 g | ad 99,30 g | ad 99,30 g |

**Tabelle 2**

| Zusammensetzung (B) | (B) |
|---|---|
| 1,6-Diaminohexan | 0,70 g |

1,50 g 5,6-Dihydroxyindolin (Hydrobromid) wurden in 90 g Wasser gelöst und durch Zugabe von Ascorbinsäure (sowie, falls notwendig, geringe Mengen Natriumhydroxid) auf den angegebenen pH-Wert eingestellt. Dann wurde mit Wasser auf 99,3 g aufgefüllt.

Kurz vor der Anwendung wurden 99,30 g der Zusammensetzung (A) mit 0,70 g der Zusammensetzung (B) vermischt. Nach dem Vermischen lagen 100 g der jeweiligen Mischung aus (A) und (B) vor.

Die Mischungen der Zusammensetzungen (A) und (B) besaßen die folgenden pH-Werte

**Tabelle 3**

| Mischung aus (A) plus (B) | (A1) + (B) | (A2) + (B) | (A3) + (B) | (A4) + (B) |
|---|---|---|---|---|
| pH-Wert | 4,2 | 6,9 | 9,5 | 9,7 |

### 2. Anwendung auf Haarsträhnen

Zur Befreiung von gegebenenfalls anhaftenden Bestandteilen (Mottenpulver, Konservierungsmittel o.ä.) wurden Haarsträhnen (Kerling Euronaturhaar weiß und Büffelbauchhaar (Yak)) mit einem handelsüblichen Shampoo (Schauma 7 Kräuter) vorgewaschen, mit Wasser ausgespült und dann getrocknet. Die so gesäuberten Strähnen wurden für mindestens 72 Stunden gelagert.

Direkt nach seiner Herstellung wurde das unter Punkt 1 beschriebene anwendungsbereite Färbemittel jeweils auf eine Haarsträhne aufgetragen (Flottenverhältnis 1,6 g Färbemittel auf 0,4 g Haarsträhne) und für 10 Minuten einwirken gelassen. Danach wurde das Färbemittel unter fließendem Wasser ausgewaschen, und die Haarsträhne wurde an der Luft getrocknet. Nach einem Zeitraum von einer Stunde wurde das Farbergebnis visuell unter der Tageslichtlampe beurteilt (Tabellen 5 und 7).

Als Vergleich wurden auch die Färbemittel (A1) bis (A4) ohne Zugabe der Zusammensetzung (B) auf den Haarsträhnen ausgefärbt. Hierfür wurden je 1,6 g das Färbemittels (A1) bis (A4) auf die Haarsträhne aufgetragen (Flottenverhältnis 1,6 g Färbemittel auf 0,4 g Haarsträhne). Danach wurde das Färbemittel unter fließendem Wasser ausgewaschen, und die Haarsträhne wurde an der Luft getrocknet. Nach einem Zeitraum von einer Stunde wurde das Farbergebnis visuell unter der Tageslichtlampe beurteilt (Ausfärbungen mit 5,6-Dihydroxyindolin allein bei verschiedenen pH-Werten, Tabellen 4 und 6).

**Tabelle 4**

| Ausfärbungen auf Euronaturhaar weiß, 5,6-DHI ohne Zugabe von 1,6-Diaminohexan | (A1) | (A2) | (A3) | (A4) |
|---|---|---|---|---|
| 1 Stunde nach der Färbung Farbintensität / Nuance | weiß - hellgrau | hellgrau | mittelgrau | mittelgrau - dunkelgrau |

**Tabelle 5**

| Ausfärbungen auf Euronaturhaar weiß, 5,6-DHI plus 1,6-Diaminohexan | (A1) + (B) | (A2) + (B) | (A3) + (B) | (A4) + (B) |
|---|---|---|---|---|
| 1 Stunde nach der Färbung Farbintensität / Nuance | mittelgrau | dunkelgrau | dunkelgrau | dunkelgrau |

**Tabelle 6**

| Ausfärbungen auf Yak Haar, 5,6-DHI ohne Zugabe von 1,6-Diaminohexan | (A1) | (A2) | (A3) | (A4) |
|---|---|---|---|---|
| 1 Stunde nach der Färbung Farbintensität / Nuance | mittelgrau | mittelgrau | dunkelgrau | dunkelgrauschwarz |

**Tabelle 7**

| Ausfärbungen auf Yak Haar, 5,6-DHI plus 1,6-Diaminohexan | (A1) + (B) | (A2) + (B) | (A3) + (B) | (A4) + (B) |
|---|---|---|---|---|
| 1 Stunde nach der Färbung Farbintensität / Nuance | dunkelgrau | dunkelgrau - schwarz | dunkelgrau - schwarz | dunkelgrau - schwarz |

Die bei verschiedenen pH-Werten durchgeführten Färbungen zeigen, dass Farbintensität, die durch Anwendung von 5,6-Dihydroxyindolin allein erhalten werden konnte, mit zunehmendem pH-Wert kontinuierlich anstieg (Tabellen 4 und 6). Die höchste Farbintensität wurde im alkalischen Bereich bei einem pH-Wert von 9,8 erzielt (Ausfärbungen von (A4)).

Durch Zugabe von 1,6-Diaminohexan wurde im Vergleich zur Ausfärbung von 5,6-Dihydroxyindolin allein eine höhere Farbintensität erzielt. Auch bei Ausfärbung dieser Kombination stieg die Farbintensität zunächst mit steigendem pH-Wert an. Die Färbung, die bei einem Misch-pH-Wert von 6,9 erhalten wurde, war intensiver als die bei pH 4,2 erhaltene Färbung (Tabellen 5 und 7, Vergleich der Ausfärbungen von (A1)+(B) und (A2)+(B)). Bei pH 6,9 war aber bereits das Maximum der Farbintensität erreicht, d.h. mit weiterer Erhöhung des pH-Wertes wurde keine noch weitere Verbesserung der Farbintensität beobachtet. Durch Ausfärbung einer Kombination von 5,6-Dihydroxyindolin (Hydrobromid) und 1,6-Diaminohexan konnte deshalb eine besonders intensive Färbung bereits im neutralen pH-Bereich erhalten werden.

Die mit dem Einsatz von alkalischen Haarfärbemitteln verbundene Haarschädigung konnte auf diesem Wege vermieden werden, ohne dass eine Verminderung der Farbintensität in Kauf genommen werden musste.

## Patentansprüche

1. Mehrkomponenten-Verpackungseinheit (Kit-of-parts) zum Färben von keratinischen Fasern, insbesondere menschlichen Haaren, umfassend getrennt voneinander konfektioniert
- einen ersten Container mit einer ersten Zusammensetzung (A), und
- einen zweiten Container mit einer zweiten Zusammensetzung (B), wobei
- die Zusammensetzung (A) in einem wasserhaltigen kosmetischen Träger mindestens ein Oxidationsfarbstoffvorprodukt enthält und einen pH-Wert von 4,0 bis 8,0 besitzt, und
- die Zusammensetzung (B) mindestens eine aliphatische Di- oder Polyaminoverbindung (DA) der Formel (I) und/oder deren Salz enthält, 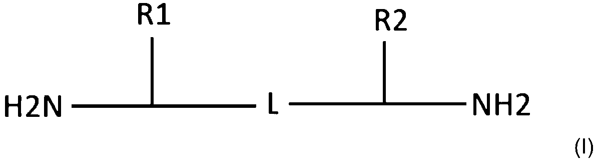
wobei
R1 und R2 unabhängig voneinander für ein Wasserstoffatom, eine C₁-C₆-Alkylgruppe oder eine Hydroxy-C₁-C₆-alkylgruppe stehen, oder aber R1 und R2 bilden zusammen mit den beiden Kohlenstoffatomen, an die sie gebunden sind, und dem Linker L einen 5-, 6- oder 7-gliedrigen, gesättigten, carbozyklischen oder heterozyklischen Ring,
L für eine direkte Bindung oder für eine gesättigte oder ungesättigte, lineare oder verzweigte C₁-C₁₂-Alkylengruppe steht, die gegebenenfalls auch mit einer oder mehreren Hydroxygruppen substituiert und/oder durch eine oder mehrere Sauerstoffatome und/oder durch eine oder mehrere Gruppierungen -NR3- unterbrochen sein kann, und
R3 für ein Wasserstoffatom, eine C₁-C₆-Alkylgruppe, eine Hydroxy-C₁-C₆-alkylgruppe oder eine Amino-C₁-C₆-Alkylgruppe steht.

2. Mehrkomponenten-Verpackungseinheit nach Anspruch 1, **dadurch gekennzeichnet, dass** die Zusammensetzung (A) als Oxidationsfarbstoffvorprodukt mindestens einen selbstoxidierenden Farbstoff (SOF) enthält, der ausgewählt ist aus der Gruppe aus 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin, 4-Aminoindolin, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol, 1,2,4-Trihydroxybenzol, Aminoresorcin, Aminohydrochinon, Diaminophenolen, 3,4,5-Trihydroxybenzoesäure sowie den physiologisch verträglichen Salzen dieser Verbindungen.

3. Mehrkomponenten-Verpackungseinheit nach einem der Ansprüche 1 bis 2, **dadurch gekennzeichnet, dass** die Zusammensetzung (A) einen pH-Wert von 4,3 bis 7,5, bevorzugt von 4,6 bis 7,3, weiter bevorzugt von 4,9 bis 7,0 und ganz besonders bevorzugt von 5,1 bis 6,5 besitzt.

4. Mehrkomponenten-Verpackungseinheit nach einem der Ansprüche 1 bis 3, **dadurch gekennzeichnet, dass** die Zusammensetzung (A) mindestens eine Säure enthält, die ausgewählt ist aus der Gruppe aus organischen und/oder anorganischen Säuren, besonders bevorzugt aus der Gruppe aus organischen Säuren, ganz besonders bevorzugt Ascorbinsäure.

5. Mehrkomponenten-Verpackungseinheit nach einem der Ansprüche 1 bis 4, **dadurch gekennzeichnet, dass** die Zusammensetzung (B) mindestens eine aliphatische Di- oder Polyaminoverbindung (DA) der Formel (I) und/oder deren Salz enthält, wobei
R1 und R2 unabhängig voneinander für ein Wasserstoffatom oder eine Methylgruppe, besonders bevorzugt beide für ein Wasserstoffatom stehen.

6. Mehrkomponenten-Verpackungseinheit nach einem der Ansprüche 1 bis 5, **dadurch gekennzeichnet, dass** die Zusammensetzung (B) mindestens eine aliphatische Di- oder Polyaminoverbindung (DA) der Formel (I) und/oder deren Salz enthält, wobei
L für eine gesättigte, lineare C₁-C₁₂-Alkylengruppe, eine gesättigte, verzweigte C₃-C₁₂-Alkylengruppe, eine Gruppierung -CH(OH)-, eine Gruppierung -CH₂-CH(OH)-CH₂-, eine Gruppierung -CH₂-O-CH₂-, eine Gruppierung -CH₂-O-CH₂-CH₂-O-CH₂-, eine Gruppierung -CH₂-O-CH₂-CH₂-O-CH₂-CH₂-O-CH₂-, eine Gruppierung -CH2-CH2-O-CH2-CH2-, eine Gruppierung -CH₂-NH-CH₂-, eine Gruppierung -CH₂-NH-CH₂-CH₂-NH-CH₂, eine Gruppierung - CH₂-NH-CH₂-CH₂-NH-CH₂-CH₂-NH-CH₂-, eine Gruppierung -CH₂-NH-CH₂-CH₂-NH-CH₂-CH₂-NH-CH₂-, eine Gruppierung -CH₂-CH₂-NR3-CH₂-CH₂- oder eine Gruppierung -CH₂-CH₂-CH₂-NR3-CH₂-CH₂-CH₂- steht.

7. Mehrkomponenten-Verpackungseinheit nach einem der Ansprüche 1 bis 6, **dadurch gekennzeichnet, dass** die Zusammensetzung (B) mindestens eine die aliphatische Di- oder Polyaminoverbindung der Formel (I) enthält, die ausgewählt ist aus der Gruppe aus 1,6-Diaminohexan, 1,5-Diaminopentan, 1,4-Diaminobutan, 1,3-Diaminopropan, 1,8-Diaminooctan, 1,10-Diamino-decan, 1,12-Diamino-dodecan, 1,5-Diamino-2-methylpentan, 1,3-Diamino-pentan, 1,3-Diamino-2,2-dimethylpropan,1,3-Diamino-2-propanol, 2-(2-Aminoethoxy)ethylamin, 2,2'-(Ethylendioxy)diethylamin, Bis(3-Aminopropyl)ether, Diethylentriamin, Triethylentetramin, Tetraethylenpentamin, Tris(3-aminopropyl)amin, Tris(2-aminoethyl)amin und deren Salzen.

8. Mehrkomponenten-Verpackungseinheit nach einem der Ansprüche 1 bis 7, **dadurch gekennzeichnet, dass** die Mischung der Zusammensetzungen (A) und (B) - bezogen auf das Gesamtgewicht der Mischung der Zusammensetzungen (A) und (B) - einen oder mehrere Oxidationsfarbstoffvorprodukte, besonders bevorzugt einen oder mehrere selbstoxidierende Farbstoffe (SOF), in einer Gesamtmenge von 0,01 bis 10,5 Gew.-%, bevorzugt von 0,1 bis 5,0 Gew.-%, weiter bevorzugt 0,2 bis 3,0 Gew.-% noch weiter besonders bevorzugt 0,3 bis 2,5 Gew.-% und ganz besonders bevorzugt von 0,6 bis 1,8 Gew.-% enthält.

9. Mehrkomponenten-Verpackungseinheit nach einem der Ansprüche 1 bis 8, **dadurch gekennzeichnet, dass** die Mischung der Zusammensetzungen (A) und (B) - bezogen auf das Gesamtgewicht der Mischung der Zusammensetzungen (A) und (B) - eine oder mehrere aliphatische Di- oder Polyaminoverbindungen (DA) der Formel (I) in einer Gesamtmenge von Gesamtmenge von 0,01 bis 20,0 Gew.-%, bevorzugt von 0,1 bis 10,0 Gew.-%, weiter bevorzugt 0,2 bis 5,0 Gew.-% noch weiter besonders bevorzugt 0,3 bis 2,5 Gew.-% und ganz besonders bevorzugt von 0,5 bis 1,0 Gew.-% enthält.

10. Mehrkomponenten-Verpackungseinheit nach einem der Ansprüche 1 bis 9, **dadurch gekennzeichnet, dass** das in der Mischung der Zusammensetzungen (A) und (B) vorhandene Gewichtsverhältnis aus allen selbstoxidierenden Farbstoffen (SOF) zu allen Di- oder Polyaminen (DA) der Formel (I), d.h. das Gewichtsverhältnis (SOF) / (DA), im Bereich von 4:1 bis 1:4, bevorzugt von 3:1 bis 1:1, weiter bevorzugt von 2,5:1 bis 1:1 und ganz besonders bevorzugt von 2,5:1 bis 1,5:1 liegt.

11. Mehrkomponenten-Verpackungseinheit nach einem der Ansprüche 1 bis 10, **dadurch gekennzeichnet, dass** die Mischung der Zusammensetzungen (A) und (B) einen pH-Wert von 4,3 bis 9,8, bevorzugt von 4,6 bis 9,0, weiter bevorzugt von 4,8 bis 8,0 und ganz besonders bevorzugt von 5,0 bis 7,5 besitzt.

12. Mehrkomponenten-Verpackungseinheit nach einem der Ansprüche 1 bis 11, **dadurch gekennzeichnet, dass** sie keine Zusammensetzung umfasst, die eine oder mehrere Peroxoverbindungen enthält.

13. Mehrkomponenten-Verpackungseinheit nach einem der Ansprüche 1 bis 12, **dadurch gekennzeichnet, dass** sie keine Zusammensetzung umfasst, die ein oder mehrere Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält.

14. Anwendungsbereites Färbemittel, das hergestellt wird durch Vermischen von
- einer Zusammensetzung (A), wie sie in den Ansprüchen 1 bis 13 definiert ist, und
- einer Zusammensetzung (B), wie sie in den Ansprüchen 1 bis 13 definiert ist.

15. Verfahren zur durch Luftsauerstoff induzierten Färbung keratinhaltiger Fasern, insbesondere menschlicher Haare, umfassend die folgenden Schritte:
- Bereitstellen einer Zusammensetzung (A), wie sie in den Ansprüchen 1 bis 13 definiert ist,
- Bereitstellen einer Zusammensetzung (B), wie sie in den Ansprüchen 1 bis 13 definiert ist,
- Herstellung eines anwendungsbereiten Färbemittels durch Vermischen der Zusammensetzungen (A) und (B),
- Auftragen der Anwendungsmischung aus (A) und (B) auf die keratinischen Fasern,
- Einwirken der auf die keratinischen Fasern aufgetragenen Mischung aus (A) und (B) auf die Fasern, und
- gegebenenfalls Ausspülen der Mischung aus (A) und (B) aus den Fasern.

16. Verfahren nach Anspruch 15, **dadurch gekennzeichnet, dass** im Verfahren
- keine Zusammensetzung auf den keratinischen Fasern angewendet wird, die eine oder mehrere Peroxoverbindungen enthält, und/oder
- keine Zusammensetzung auf den keratinischen Fasern angewendet wird, die ein Alkalisierungsmittel aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol und 1-Amino-2-methylpropan-2-ol, 1-Amino-2-methylpropan-2-ol, 3-Aminopropan-1,2-diol, 2-Amino-2-methylpropan-1,3-diol, Triethanolamin, Arginin, Lysin, Histidin und Ornithin enthält.
